# EUROPEAN PATENT SPECIFICATION

(11) **EP 3 415 198 B1**
(45) Date of publication and mention of the grant of the patent: **13.05.2020**
(21) Application number: 18176696.5
(22) Date of filing: 08.06.2018
(51) Int. Cl.: A61N 1/40

(54) **BEAUTY APPARATUS**
SCHÖNHEITSVORRICHTUNG
APPAREIL DE SOINS DE BEAUTÉ

(30) Priority: 13.06.2017 JP 2017115825
(43) Date of publication of application: 19.12.2018
(73) Proprietor: Panasonic Intellectual Property Management Co., Ltd., Osaka-shi, Osaka 540-6207 (JP)
(72) Inventor: MATSUSAKA, Takeshi, Osaka-shi, Osaka 540-6207 (JP); WATANABE, Shunichi, Osaka-shi, Osaka 540-6207 (JP); IKADAI, Kazuyasu, Osaka-shi, Osaka 540-6207 (JP); FUJIWARA, Mitsuru, Osaka-shi, Osaka 540-6207 (JP)
(74) Representative: Grünecker Patent- und Rechtsanwälte PartG mbB

(56) References cited:
- WO-A1-2014/147855
- WO-A1-2016/087289
- US-B1- 6 413 255

## Description

### BACKGROUND

### 1. Technical Field

The present disclosure relates to a beauty apparatus used for a skin.

### 2. Description of the Related Art

In the related art, a configuration of a beauty apparatus is known which can obtain a beautiful skin effect by applying an ultrasound wave to a skin as disclosed in Japanese Patent No. 4416028, for example.

### SUMMARY

In recent years, there is a demand on a beauty apparatus which achieves a further improved beautiful skin effect.

Therefore, the present disclosure aims to provide a beauty apparatus which can further improve a beautiful skin effect by providing a skin with different stimulations.

According to an aspect of the present disclosure, there is provided a beauty apparatus including a first stimulation provider that provides a skin with a first stimulation, and a second stimulation provider that provides the skin with a second stimulation which is a stimulation induced by a Radio Frequency (RF) and different from the first stimulation. The beauty apparatus further includes a housing that holds the first stimulation provider and the second stimulation provider, the second stimulation provider being located in the vicinity of the first stimulation provider.

According to this configuration, it is possible to provide the beauty apparatus which can provide the skin with different stimulations and which can further improve a beautiful skin effect.

The invention is defined by claim 1. Preferred embodiments are defined by the dependent claims. Further embodiments, aspects and examples are disclosed for exemplary purpose only and do not form part of the invention as claimed.

### BRIEF DESCRIPTION OF THE DRAWINGS

FIG. 1 is a plan view illustrating a schematic configuration of a beauty apparatus according to an exemplary embodiment;
FIG. 2 is a sectional view taken along line 2-2 in FIG. 1;
FIG. 3 is a plan view illustrating a schematic configuration of a head of the beauty apparatus according to the exemplary embodiment;
FIG. 4 is a sectional view taken along line 4-4 in FIG. 3;
FIG. 5 is a perspective view illustrating a schematic configuration of a second holder and a second stimulation provider of the beauty apparatus according to the exemplary embodiment;
FIG. 6 is a perspective view illustrating a state where a portion of the second holder is detached from the beauty apparatus according to the exemplary embodiment;
FIG. 7 is a perspective view illustrating a schematic configuration of a terminal of the beauty apparatus according to the exemplary embodiment;
FIG. 8 is a perspective view illustrating a schematic configuration of a conductive plate of the beauty apparatus according to the exemplary embodiment;
FIG. 9 is a sectional view illustrating a state where a protruding portion of a probe head of the beauty apparatus according to the exemplary embodiment is pressed against a skin;
FIG. 10 is a graph illustrating a relationship between skin temperature of a skin and skin impedance;
FIG. 11 is a graph illustrating an evaluation result of a lifted feeling of the skin depending on whether or not a flat portion is present;
FIG. 12 is a view for describing examples of a layout of an RF electrode of a beauty apparatus according to modification examples;
FIG. 13 is a graph illustrating a relationship between a time required until a user feels warm and W-density of the RF electrode; and
FIG. 14 is a graph illustrating a relationship between the time required until the user feels warm and the number of electrodes.

### DETAILED DESCRIPTION

Hereinafter, a beauty apparatus according to exemplary embodiments of the present disclosure will be described in detail with reference to the drawings. All of the exemplary embodiments described below are preferred specific examples of the present disclosure. Accordingly, numerical values, shapes, materials, configuration elements, or arrangements and connecting forms of the configuration elements, which are illustrated in the following exemplary embodiments, are merely examples, and do not limit the present disclosure. Therefore, in the configuration elements according to the following exemplary embodiment, the configuration elements which are not described in an independent claim representing a most significant concept of the present disclosure will be described as optional configuration elements.

Each drawing is schematically illustrated, and is not necessarily strictly illustrated. In each drawing, the same reference numerals are given to the same configuration members.

### Beauty Apparatus

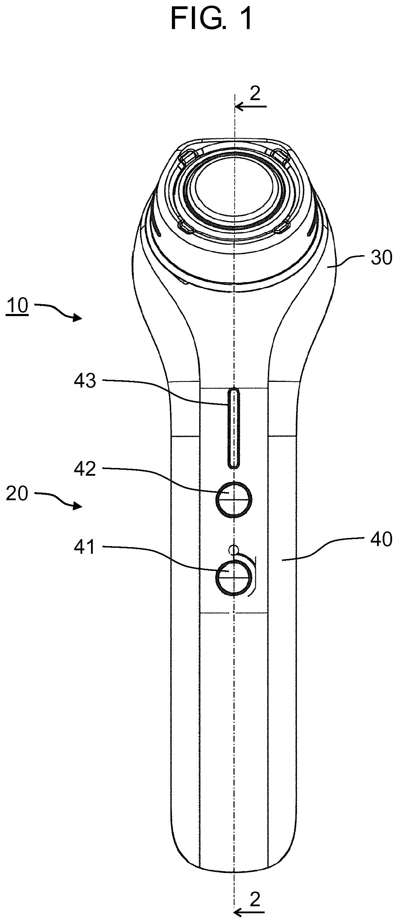

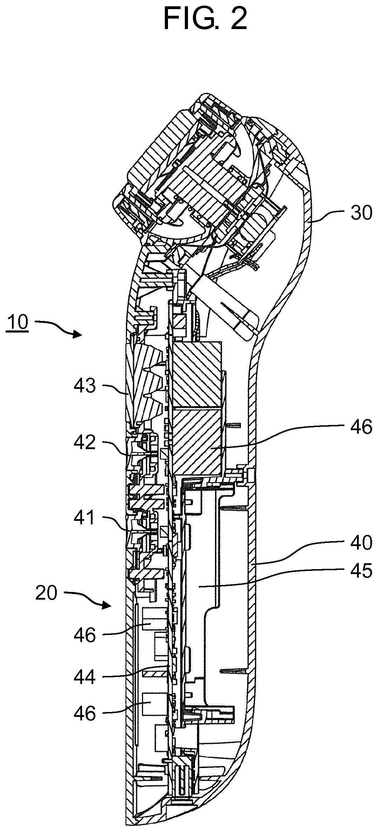

First, a beauty apparatus according to an exemplary embodiment will be described. FIG. 1 is a plan view illustrating a schematic configuration of the beauty apparatus according to an exemplary embodiment. FIG. 2 is a sectional view taken along line 2-2 in FIG. 1.

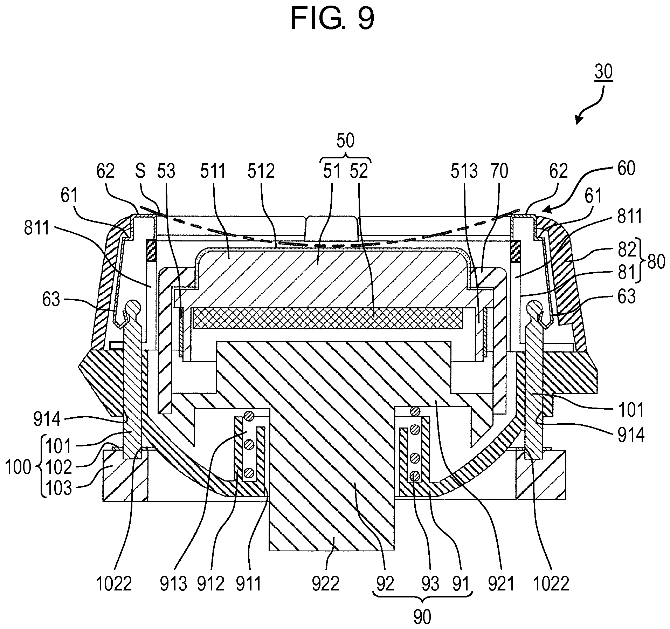

As illustrated in FIGS. 1 and 2, beauty apparatus 10 includes elongated housing 20 which is an external body. One end portion of housing 20 serves as head 30 that provides skin S with a stimulation (refer to FIG. 9). A part other than head 30 in housing 20 is gripper 40 gripped by a user, and is formed narrower than head 30. Gripper 40 has power button 41 for switching on and off power, selection button 42 for selecting an operation mode of beauty apparatus 10, and lighting portion 43 which is turned on when the power is switched on and is turned off when the power is switched off.

As illustrated in FIG. 2, circuit board 44 and battery 45 are accommodated inside gripper 40. A plurality of circuit components 46 for driving a drive unit of head 30 are mounted on circuit board 44. The plurality of circuit components 46 include a microcomputer which controls the drive unit of head 30. The plurality of circuit components 46 include a pair of switch elements. One of the pair of switch elements is used in order to turn on and off power. Power button 41 is pressed down so as to switch on and off power supply from battery 45. The other switch element is used in order to select the operation mode. Selection button 42 is pressed down so as to output a control signal corresponding to each operation mode to the microcomputer.

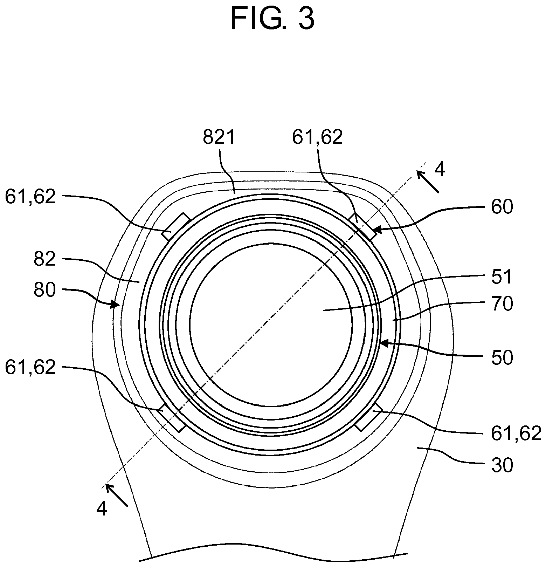

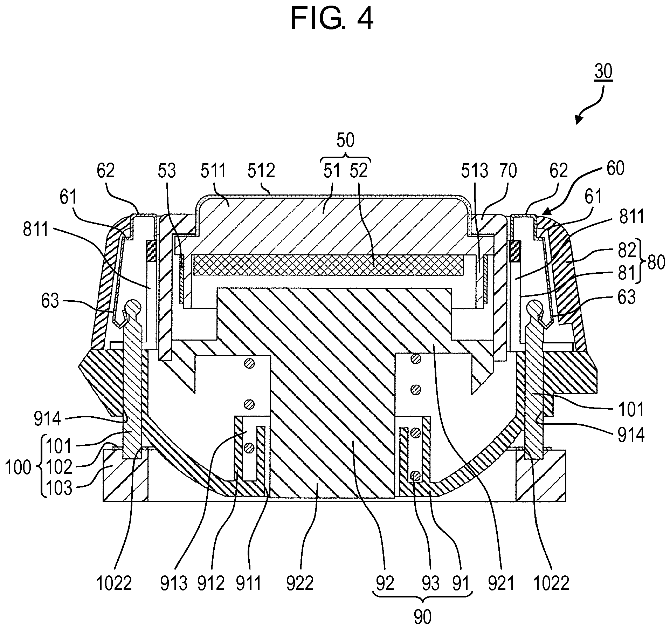

FIG. 3 is a plan view illustrating a schematic configuration of the head of the beauty apparatus according to the exemplary embodiment. FIG. 4 is a sectional view taken along line 4-4 in FIG. 3.

As illustrated in FIGS. 3 and 4, head 30 includes first stimulation provider 50, second stimulation provider 60, first holder 70, second holder 80, retractable mechanism 90, and terminal 100. First holder 70 and second holder 80 hold first stimulation provider 50 and second stimulation provider 60, second stimulation provider 60 being located in the vicinity of first stimulation provider 50. In this manner, first stimulation provider 50 and second stimulation provider 60 can simultaneously come into contact with skin S (refer to FIG. 9) of a user.

First stimulation provider 50 provides skin S with a first stimulation. Examples of the first stimulation include an ultrasound wave vibration stimulation, an electrical stimulation (for example, an electrical muscle stimulation (EMS)), a thermal stimulation, and light stimulation. In this exemplary embodiment, the ultrasound wave vibration stimulation will be described as an example of the first stimulation.

First stimulation provider 50 includes probe head 51 and vibrator 52 which vibrates probe head 51. Probe head 51 is a metal body which comes into contact with skin S and transmits vibration generated by vibrator 52 to skin S with which probe head 51 is in contact. In probe head 51, a portion exposed from head 30 protrudes in a disc shape. A surface of protruding portion 511 serves as oxide film 512. For example, in this exemplary embodiment, after probe head 51 is formed of aluminum, protruding portion 511 is subjected to alumite processing so as to produce oxide film 512 formed of aluminum oxide. The surface of probe head 51 may entirely serve as oxide film 512.

Circularly erected peripheral wall 513 is formed on a side opposite to protruding portion 511 in probe head 51. Sheet-like heater 53 is attached to an entire outer peripheral surface of peripheral wall 513. Heater 53 heats skin S via probe head 51 by heating probe head 51. Heater 53 is electrically connected to the microcomputer, and temperature thereof is adjusted under the control of the microcomputer.

Vibrator 52 vibrates probe head 51 by vibrating at a frequency bandwidth of the ultrasound wave, and applies the ultrasound wave vibration stimulation to skin S in contact with probe head 51. Specifically, vibrator 52 is a piezoelectric element, and is fixed to probe head 51 inside a region surrounded by peripheral wall 513. In vibrator 52, the vibration is controlled under the control of the microcomputer.

First holder 70 is a resin member which holds first stimulation provider 50. For example, first holder 70 is formed in a substantially cylindrical shape. First holder 70 is attached to an outer peripheral edge of protruding portion 511, protruding portion 511 of probe head 51 protruding outward from a center of one end portion thereof. First holder 70 is connected to retractable mechanism 90.

In retractable mechanism 90, first holder 70 is retracted from head 30. In this manner, first stimulation provider 50 is retracted from second stimulation provider 60. Specifically, retractable mechanism 90 includes base 91, connector 92, and elastic member 93.

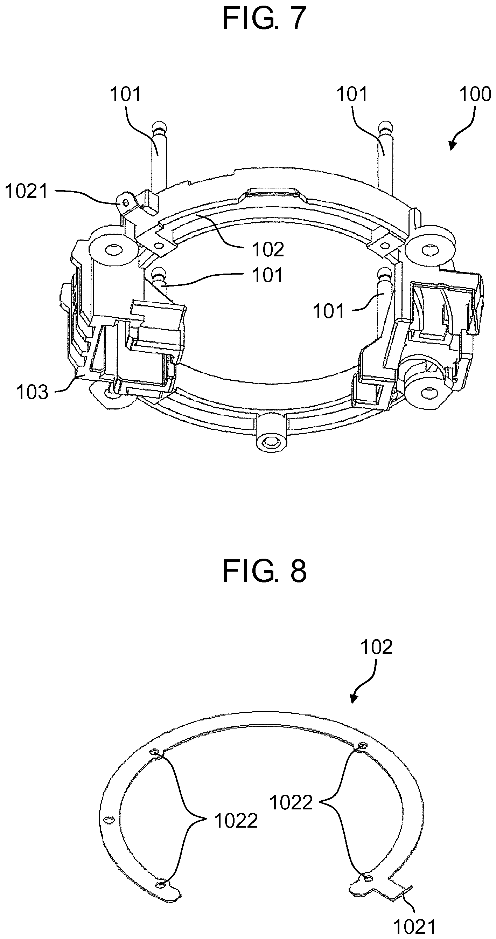

Base 91 is a resin member configuring a portion of head 30, and is formed in a substantial cup shape. Connector 92 relatively moves to or from base 91. Specifically, through-hole 911 is formed at the center of base 91, and tubular portion 912 concentric with through-hole 911 is formed around through-hole 911. Tubular portion 912 has accommodation groove 913 which accommodates elastic member 93. Accommodation groove 913 is formed concentrically with through-hole 911. A plurality of attachment holes 914 are formed on an outer peripheral portion of base 91. A plurality of electrode pins 101 included in terminal 100 are separately attached to the plurality of attachment holes 914 (refer to FIG. 7).

Connector 92 is a resin member connected to first holder 70. Connector 92 includes fitting portion 921 fitted to first holder 70 and shaft portion 922 protruding in a direction from fitting portion 921 to base 91 and inserted into through-hole 911 of base 91.

When elastically deformed, elastic member 93 applies a biasing force so that head 30 is biased against connector 92 in an outward direction. Specifically, elastic member 93 is a coil spring. One end portion thereof is fixed into accommodation groove 913 of base 91, and the other end portion is fixed to fitting portion 921 of connector 92. That is, in a state where elastic member 93 is accommodated in accommodation groove 913, elastic member 93 is stretched by being elastically deformed / elastically restored. Elastic member 93 may be a spring other than the coil spring, or may be other elastic bodies such as rubber. A retracting operation performed by retractable mechanism 90 will be described later.

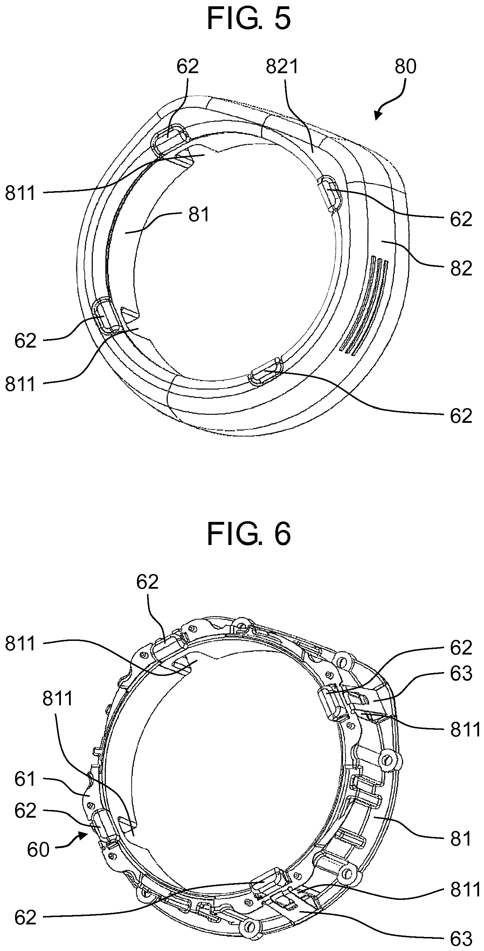

FIG. 5 is a perspective view illustrating a schematic configuration of the second holder and the second stimulation provider of the beauty apparatus according to the exemplary embodiment. FIG. 6 is a perspective view illustrating a state where a portion of the second holder is detached from the beauty apparatus according to the exemplary embodiment.

As illustrated in FIGS. 4 to 6, second stimulation provider 60 is integrally assembled with second holder 80, and in a state of being assembled, second holder 80 is detachable from first holder 70. Specifically, second holder 80 is detachable from base 91 of retractable mechanism 90 to which first holder 70 is connected.

Second stimulation provider 60 provides skin S (refer to FIG. 9) with a stimulation induced by a radio frequency (hereinafter, referred to as an "RF"), which is the second stimulation different from the first stimulation provided by first stimulation provider 50. Examples of the second stimulation include a stimulation for heating a deep portion of skin S by using the RF so as to generate an electric field or a magnetic field on a surface or an inner portion of skin S. In this exemplary embodiment, as an example of the second stimulation, a stimulation induced by the electric field generated by the RF will be described.

Specifically, second stimulation provider 60 includes RF electrode 61 formed in an annular shape. RF electrode 61 is formed of conductive metal. For example, in this exemplary embodiment, RF electrode 61 is formed of titanium. RF electrode 61 has a plurality of projections 62 protruding from second holder 80. The plurality of projections 62 are arranged at an equal interval in a circumferential direction. In this exemplary embodiment, as illustrated in FIGS. 3 to 5, a case has been described as an example having four projections 62 in which a shape of the portion exposed from second holder 80 is rectangular in a plan view. However, the shape and the number of projections 62 may be optionally set.

As illustrated in FIGS. 4 to 6, a portion facing projection 62 in RF electrode 61 has locking piece 63 extending toward a side opposite to projection 62. Therefore, RF electrode 61 includes locking pieces 63 whose number is the same as the number of the plurality of projections 62. Locking piece 63 is formed in a plate spring shape whose tip end portion is bent. Electrode pin 101 of terminal 100 is locked to the tip end portion of locking piece 63.

In a case where second stimulation provider 60 provides the stimulation induced by the electric field generated by the RF, it is necessary to provide the other RF electrode having a polarity different from that of RF electrode 61. In this exemplary embodiment, probe head 51 of first stimulation provider 50 also serves as the other RF electrode. In this manner, as a whole, head 30 includes a pair of RF electrodes (RF electrode 61 and probe head 51) having different polarities.

Here, the RF is an electromagnetic wave having a frequency bandwidth from 10 kHz to 100 GHz. From a viewpoint of a cosmetic effect, it is further desirable that the RF is an electromagnetic wave or a current having a frequency bandwidth from 0.3 MHz to 50 MHz. As the RF, this exemplary embodiment employs a current of approximately 1 MHz which is suitable for aluminum-made probe head 51 provided with oxide film 512.

Second holder 80 is a member freely detachable from first holder 70 and holding second stimulation provider 60. Specifically, as a whole, second holder 80 has an annular outer shape, and first stimulation provider 50 is located inside an opening portion of second holder 80. Second holder 80 includes frame 81 and cover 82. Frame 81 is an annularly formed resin member, and holds RF electrode 61 of second stimulation provider 60. Frame 81 has guide route 811 which guides each locking piece 63 of RF electrode 61 to electrode pin 101 of terminal 100. Cover 82 is an annularly formed resin member, and is attached to frame 81 so as to cover a portion other than projection 62 of RF electrode 61 in a state where projection 62 of RF electrode 61 is individually exposed. A portion of an edge portion of cover 82 is formed in a flat shape. The portion formed in the flat shape will be referred to as flat portion 821. Flat portion 821 is located in a tip end portion of housing 20, and extends in a direction orthogonal to a longitudinal direction of housing 20. For example, in a case where an opening portion of cover 82 has a circular shape, flat portion 821 is formed in a flat shape substantially parallel to a tangent line of the opening portion. In this manner, flat portion 821 in cover 82 is located closer to first stimulation provider 50 and second stimulation provider 60 than a portion other than flat portion 821 in cover 82. Specifically, as illustrated in FIG. 3, flat portion 821 as a whole surrounds a periphery of probe head 51 of first stimulation provider 50. Flat portion 821 surrounds a periphery of projection 62 of second stimulation provider 60.

FIG. 7 is a perspective view illustrating a schematic configuration of terminal 100 of the beauty apparatus according to the exemplary embodiment.

As illustrated in FIGS. 4 and 7, second stimulation provider 60 is electrically connected to terminal 100 so as to supply power to second stimulation provider 60. Specifically, terminal 100 includes the plurality of electrode pins 101, conductive plate 102, and bracket 103.

Electrode pin 101 is formed in a columnar shape, and a tip end portion thereof is formed in a substantially spherical shape. Electrode pin 101 is attached to base 91. Electrode pin 101 is inserted into and fixed to attachment hole 914 of base 91, and the tip end portion of electrode pin 101 is in a state of protruding from attachment hole 914. Locking piece 63 of RF electrode 61 is locked to the tip end portion of electrode pin 101, thereby bringing second holder 80 into a state where second holder 80 is attached to first holder 70 (refer to FIG. 4). In this state, RF electrode 61 and electrode pin 101 are electrically connected to each other.

On the other hand, if second holder 80 is pulled in a direction away from first holder 70, that is, in an upward direction in FIG. 4, the tip end portion of electrode pin 101 and locking piece 63 are unlocked from each other, and second holder 80 is brought into a state where second holder 80 is detached from first holder 70. In this state, RF electrode 61 and electrode pin 101 are electrically connected to each other.

FIG. 8 is a perspective view illustrating a schematic configuration of conductive plate 102 configuring terminal 100 of the beauty apparatus according to the exemplary embodiment. FIG. 8 is a perspective view in a case where conductive plate 102 is viewed in a direction different from that of FIG. 7. As illustrated in FIG. 8, conductive plate 102 is a conductive metal plate formed in a substantial C-shape. Tongue portion 1021 protruding outward is formed in one end portion of conductive plate 102. Tongue portion 1021 is electrically connected to power supplying circuit component 46 (refer to FIG. 2) via a wire (not illustrated). A C-shaped portion of conductive plate 102 has a plurality of connection holes 1022 to which the plurality of electrode pins 101 are individually connected. If electrode pin 101 is connected to connection hole 1022, conductive plate 102 and electrode pin 101 are electrically connected to each other. As described above, the plurality of electrode pins 101 are electrically connected to RF electrode 61 of second stimulation provider 60. Accordingly, conductive plate 102 and RF electrode 61 are electrically connected to each other via electrode pins 101. That is, terminal 100 and second stimulation provider 60 are electrically connected to each other at a plurality of locations. Accordingly, even if one of electrode pins 101 is broken and is not in an electrically connected state, the electrically connected state is maintained by other remaining electrode pins 101.

As illustrated in FIG. 7, bracket 103 is a member which holds the plurality of electrode pins 101 and conductive plate 102. Bracket 103 is formed in an annular shape, and a portion of retractable mechanism 90 is located inside an opening portion of bracket 103 (refer to FIG. 4). Bracket 103 is fixed into housing 20.

### Operation

Next, an operation of beauty apparatus 10 will be described.

First, a user applies moisture-containing cosmetics or treatment gels to skin S (refer to FIG. 9) serving as a treatment target and at least one probe heads 51 of beauty apparatus 10. Thereafter, if the user operates power button 41, beauty apparatus 10 is in a state where power is turned on. Next, if the user operates selection button 42, the microcomputer controls each drive unit in an operation mode desired by the user. Specifically, the microcomputer controls vibrator 52 so as to apply a predetermined ultrasound wave vibration to probe head 51. The microcomputer supplies a voltage having the frequency bandwidth which generates the RF to probe head 51 and RF electrode 61. The microcomputer controls heater 53 so as to heat heater 53 up to a predetermined temperature.

Immediately after the power is supplied, as illustrated in FIG. 4, elastic member 93 of retractable mechanism 90 is in a stretched state, and protruding portion 511 of probe head 51 protrudes from head 30. Thereafter, the user then presses protruding portion 511 of probe head 51 against skin S.

FIG. 9 is a sectional view illustrating a state where the protruding portion of the probe head of the beauty apparatus according to the exemplary embodiment is pressed against the skin. Specifically, FIG. 9 corresponds to FIG. 4. In FIG. 9, an outline of skin S is illustrated by a two-dot chain line.

If protruding portion 511 of probe head 51 is pressed against skin S in a state illustrated in FIG. 4, probe head 51 descends from head 30, and elastic member 93 is contracted as illustrated in FIG. 9. In this state, probe head 51 is hidden inside head 30. In this manner, protruding portion 511 of probe head 51 and projection 62 of RF electrode 61 located on a side of skin S from probe head 51 due to probe head 51 descending from head 30 come into contact with skin S. At this time, three stimulations are applied to skin S.

The three stimulations will be specifically described.

First, the first stimulation is the ultrasound wave vibration induced by vibrator 52. The ultrasound wave vibration generated by vibrator 52 is applied to skin S via probe head 51. The ultrasound wave vibration is applied to skin S, thereby allowing moisture distribution inside skin S to be uniform. Accordingly, tension can be applied to skin S.

Next, the second stimulation is the RF induced by the pair of RF electrodes (probe head 51 and RF electrode 61). If both probe head 51 and RF electrode 61 come into contact with skin S in a state where a voltage having the frequency bandwidth which generates the RF is applied, an electric field is generated at a specific region of skin S, and Joule heat generated by the current is generated in a portion where the electric field is generated. In this way, if the specific region of skin S is heated, skin S can be activated by promoted blood flow and action of gene expression.

The third stimulation is heat induced by heater 53. If heater 53 is heated, probe head 51 in contact with heater 53 is also heated. In this manner, the heat is transferred from probe head 51 to skin S, and skin S becomes warm.

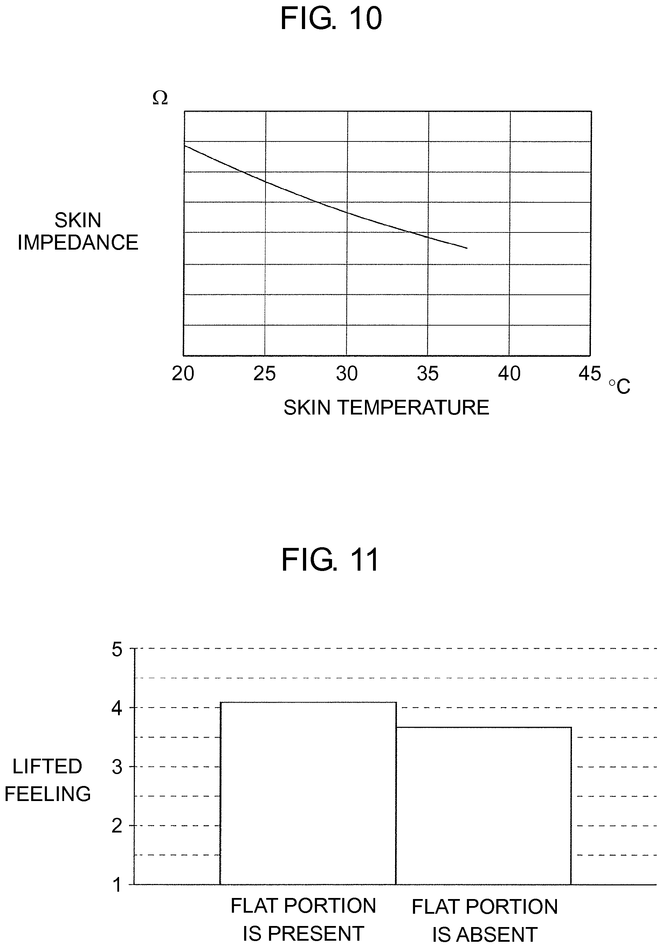

FIG. 10 is a graph illustrating a relationship between skin temperature of skin S and skin impedance. As illustrated in FIG. 10, if the skin temperature rises, the skin impedance tends to decrease. That is, since the skin temperature rises, an electric current is likely to flow inside skin S. Accordingly, the electric field induced by the RF is likely to be generated inside skin S, and the electric current is likely to flow. Therefore, the deep portion of skin S is more likely to be heated, a heating effect achieved using the RF can be improved.

In a state where probe head 51 is pressed against skin S, the user moves beauty apparatus 10 so that probe head 51 slides on skin S. In this case, if beauty apparatus 10 is moved in a direction where flat portion 821 moves forward, the user can obtain a more lifted feeling.

FIG. 11 is a graph illustrating an evaluation result of the lifted feeling of skin S depending on whether or not flat portion 821 is present. In a case where flat portion 821 is not present, the whole edge portion of cover 82 is set to have a circular shape in a plan view. The lifted feeling is determined by each user through a five-stage evaluation (lifted feeling 1 is "not satisfactory" and lifted feeling 5 is "satisfactory"), and an average value thereof is illustrated. As illustrated in Fig. 11, it is understood that the evaluation of the lifted feeling is improved by 10% or more in a case where flat portion 821 is present, compared to a case where flat portion 821 is not present. In flat portion 821, probe head 51 of first stimulation provider 50 and projection 62 of second stimulation provider 60 are located closer than the other portions in cover 82. Accordingly, probe head 51 and projection 62 are likely to touch an intended place of the user.

Thereafter, if the user operates probe head 51 so that skin S is no longer in contact with probe head 51 in a state illustrated in FIG. 9, elastic member 93 is elastically restored to stretch as illustrated in FIG. 4, and probe head 51 returns to the original position. In this state, probe head 51 protrudes from head 30. In this way, retractable mechanism 90 enables probe head 51 to retract with respect to head 30.

### Advantageous Effect

As described above, beauty apparatus 10 according to this exemplary embodiment includes first stimulation provider 50 that provides skin S with the first stimulation, and second stimulation provider 60 that provides skin S with the second stimulation which is the stimulation induced by the RF and different from the first stimulation. Beauty apparatus 10 further includes housing 20 that holds first stimulation provider 50 and second stimulation provider 60, second stimulation provider 60 being located in the vicinity of first stimulation provider 50.

According to this configuration, housing 20 allows second stimulation provider 60 to be located in the vicinity of first stimulation provider 50. Accordingly, the first stimulation and the second stimulation can be simultaneously provided for skin S. In this manner, skin S can be provided with different stimulations, and the beautiful skin effect can be further improved.

Second stimulation provider 60 includes one RF electrode 61 of the pair of RF electrodes, and first stimulation provider 50 also serves as the other RF electrode of the pair of RF electrodes.

According to this configuration, second stimulation provider 60 includes one RF electrode 61 of the pair of RF electrodes, and first stimulation provider 50 also serves as the other RF electrode of the pair of RF electrodes. Accordingly, the pair of RF electrodes can generate the electric field in the deep portion of skin S so that the current generates Joule heat.

First stimulation provider 50 also serves as the other RF electrode. Accordingly, compared to a case where each of the pair of RF electrodes is prepared as a dedicated member, the number of components can be reduced, and head 30 can have a compact size. If head 30 has the contact size, operability during treatment is improved.

At least one (probe head 51) of the pair of RF electrodes is a metal body whose surface has oxide film 512, and the pair of RF electrodes is supplied with a voltage having the frequency bandwidth of 0.3 MHz or higher.

According to this configuration, at least one (probe head 51) of the pair of RF electrodes has oxide film 512 on the surface. Accordingly, it is possible to prevent probe head 51 from being rusted. The pair of RF electrodes is supplied with the voltage having the frequency bandwidth of 0.3 MHz or higher. Accordingly, even if oxide film 512 is present, the pair of RF electrodes can be electrically connected to each other.

Housing 20 fixes one of first stimulation provider 50 and second stimulation provider 60. Beauty apparatus 10 includes retractable mechanism 90 which causes the other of first stimulation provider 50 and second stimulation provider 60 to retract with respect to the one of first stimulation provider 50 and second stimulation provider 60.

According to this configuration, the other one retracts with respect to the fixed one of first stimulation provider 50 and second stimulation provider 60. Accordingly, both first stimulation provider 50 and second stimulation provider 60 are reliably allowed to be in close contact with skin S. In this manner, during the treatment, the first stimulation and the second stimulation reliably act on skin S. Therefore, the beautiful skin effect can be further improved.

Housing 20 includes first holder 70 and second holder 80, first holder 70 holding first stimulation provider 50, second holder 80 holding second stimulation provider 60 and being freely detachable from first holder 70..

Here, moisture-containing cosmetics or gels are used during the treatment. Accordingly, it is necessary to clean first stimulation provider 50 and second stimulation provider 60 after the treatment. Cosmetics or gels permeate into even a small gap, thereby causing the cleaning to be complicated. However, if second holder 80 is freely detachable from first holder 70, first stimulation provider 50 and second stimulation provider 60 can be easily cleaned. Since second holder 80 is freely detachable from first holder 70, replacement work can be easily carried out.

Second holder 80 has an annular shape and surrounds first stimulation provider 50, and a portion of the edge portion of second holder 80 is formed in a flat shape.

According to this configuration, since the portion of the edge portion of second holder 80 is formed in the flat shape, the lifted feeling for skin S can be improved.

Beauty apparatus 10 further includes terminal 100 which supplies the power to second stimulation provider 60, and second stimulation provider 60 and terminal 100 are electrically connected to each other at the plurality of locations.

According to this configuration, terminal 100 and second stimulation provider 60 are electrically connected to each other at the plurality of locations. Therefore, even if one connecting place is brought into a non-electrically connected state, the electrically connected state can be maintained by the other connecting place.

Beauty apparatus 10 further includes heater 53 which heats skin S from the surface.

According to this configuration, heater 53 can heat skin S from the surface. Since the skin temperature of skin S rises, the electric current is likely to flow inside skin S. Accordingly, the electric field induced by the RF is likely to be generated inside skin S. Therefore, the inner side of skin S is likely to be heated, and the heating effect achieved by the RF can be improved.

### Modification Examples

In the above-described exemplary embodiment, a case has been described as an example where four projections 62 of RF electrode 61 are arranged at an equal interval around protruding portion 511 of probe head 51. However, the shape and the layout of the RF electrode may be optionally determined as long as the projection of the RF electrode is located in the vicinity of protruding portion 511 of probe head 51. Here, other examples of the layout of the RF electrode will be described.

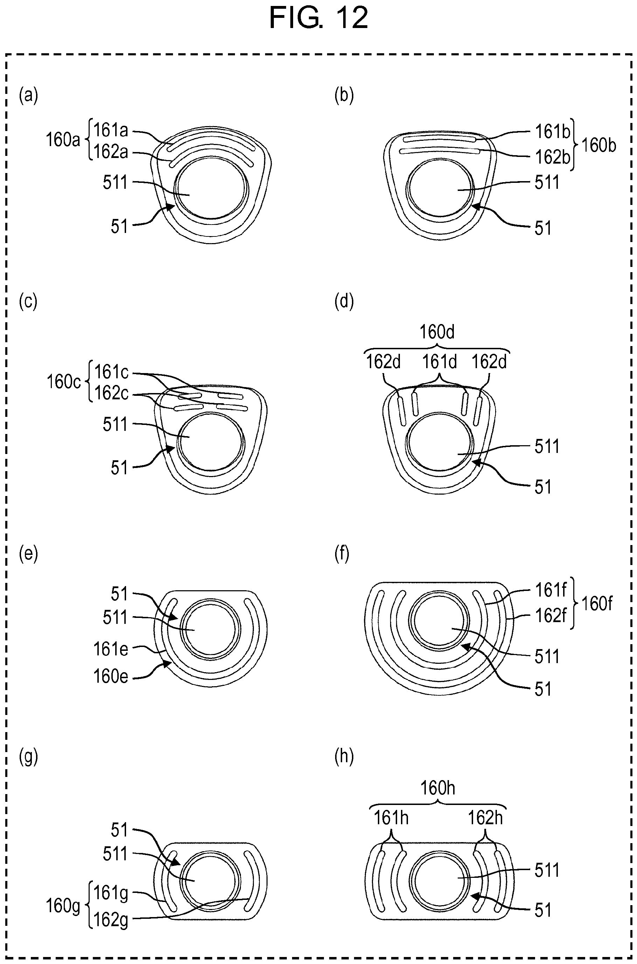

FIG. 12 is a view for describing the examples of the layout of the RF electrode according to modification examples. In the layout illustrated in (a) of FIG. 12, double arc-shaped projections 161a and 162a of RF electrode 160a are arranged in the vicinity of protruding portion 511 of probe head 51. Double arc-shaped projections 161a and 162a are curved along the outer peripheral edge of protruding portion 511.

In the layout illustrated in (b) of FIG. 12, double linear projections 161b and 162b of RF electrode 160b are arranged in the vicinity of protruding portion 511 of probe head 51. Double linear projections 161b and 162b are straight lines along the outer peripheral edge of protruding portion 511.

In the layout illustrated in (c) of FIG. 12, two sets of double linear projections 161c and 162c of RF electrode 160c are arranged in the vicinity of protruding portion 511 of probe head 51. Double linear projections 161c and 162c of each set are straight lines along the outer peripheral edge of protruding portion 511.

In the layout illustrated in (d) of FIG. 12, two sets of double linear projections 161d and 162d of RF electrode 160d are arranged in the vicinity of protruding portion 511 of probe head 51. Double linear projections 161d and 162d of each set are straight lines along a direction intersecting the outer peripheral edge of protruding portion 511.

In the layout illustrated in (e) of FIG. 12, arc-shaped projection 161e of RF electrode 160e is located in the vicinity of protruding portion 511 of probe head 51. Projection 161e is formed in an arc shape surrounding a most portion of protruding portion 511.

In the layout illustrated in (f) of FIG. 12, double arc-shaped projections 161f and 162f of RF electrode 160f are arranged in the vicinity of protruding portion 511 of probe head 51. Double arc-shaped projections 161f and 162f are formed in an arc shape surrounding the most portion of protruding portion 511.

In the layout illustrated in (g) of FIG. 12, a pair of arc-shaped projections 161g and 162g of RF electrode 160g is arranged in the vicinity of protruding portion 511 of probe head 51. The pair of projections 161g and 162g face each other across protruding portion 511.

In the layout illustrated in (h) of FIG. 12, two sets of double arc-shaped projections 161h and 162h of RF electrode 160h are arranged in the vicinity of protruding portion 511 of probe head 51. In double arc-shaped projections 161h and 162h, the respective sets face each other across protruding portion 511.

In the above-described respective modification examples, probe head 51 also serves as the RF electrode. A dedicated member of the pair of RF electrodes may form each of the plurality of projections. In this case, the second stimulation provider includes the pair of RF electrodes. The pair of RF electrodes can generate the electric field inside skin S so that the current generates Joule heat. In this case, probe head 51 can be used as a dedicated member for the ultrasound wave vibration.

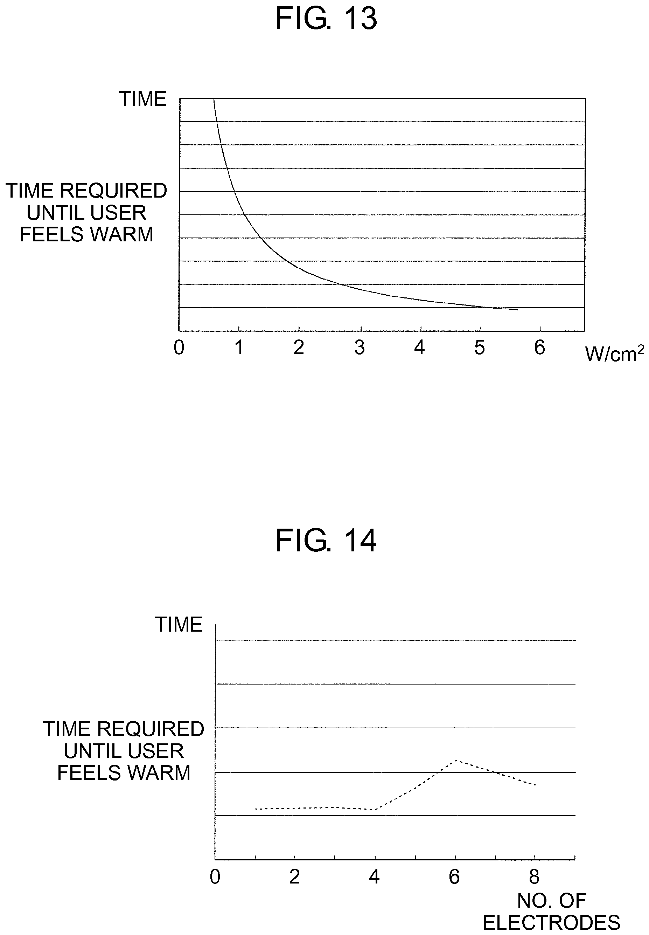

FIG. 13 is a graph illustrating a relationship between a time required until a user feels warm and W-density of the RF electrode.
Specifically, in a state where the power is kept constant, the W-density of the RF electrode is changed so as to measure the time required until the user feels warm. A result thereof is illustrated by the graph in FIG. 13. As illustrated in FIG. 13, until the W-density is lower than approximately 3 W/cm², the time required until the user feels warm greatly decreases. However, subsequently, the time is smoothly changed. Based on this result, the W-density of the RF electrode may be approximately 3 W/cm² or higher.

FIG. 14 is a graph illustrating a relationship between the time required until the user feels warm and the number of electrodes. Specifically, the whole exposed area in the RF electrode in contact with skin S is kept constant, and the number of electrodes (number of the projections of the RF electrode) is changed. That is, if the number of electrodes increases, the area in one RF electrode in contact with skin S decreases. For each number of electrodes, the time required until the user feels warm is measured. A result thereof is illustrated by the graph in FIG. 14. As illustrated in FIG. 14, in a case where the number of electrodes is four, the time required until the user feels warm is faster. Therefore, the number of electrodes may be four. In view of the relationships illustrated in FIGS. 13 and 14, it is desirable to examine the layout, the shape, or the material of the RF electrode.

### Alternatives

Hitherto, the beauty apparatus according to the present disclosure has been described with reference to the above-described exemplary embodiment and modification examples. However, the present disclosure is not limited to the above-described exemplary embodiment and modification examples.

For example, in the above-described exemplary embodiment, a case has been described as an example where probe head 51 also serving as one of the pair of RF electrodes is formed of aluminum. However, probe head 51 may be formed of another metal. As another metal, stainless steel or titanium may be used. Even in a case of using another metal, it is preferable to produce the oxide film on the surface.

In this exemplary embodiment, a case has been described as an example where probe head 51 of first stimulation provider 50 also serves as one of the pair of RF electrodes. However, a dedicated member serving as the RF electrode may be provided. Even in a case of using the dedicated member serving as the RF electrode, it is preferable to produce the oxide film on the surface. If the oxide film is provided, electrical conductivity is degraded. However, if the frequency bandwidth of the voltage supplied to the pair of RF electrodes is set to 0.3 MHz or higher, even if the oxide film is present, constant electrical conductivity can be ensured.

In the above-described exemplary embodiment, a case has been described as an example where the electric field is generated from the surface to the inner side of skin S by the pair of RF electrodes so that the current generates Joule heat. However, the second stimulation provider can cause one of the RF electrodes to generate a magnetic field in skin S. In this manner, the magnetic field can provide skin S with a thermal effect and blood circulation promoting action. That is, the second stimulation in this case induces the magnetic field.

In the above-described exemplary embodiment, a case has been described as an example where one of the RF electrodes (probe head 51) of the pair of RF electrodes has oxide film 512 and other RF electrode 61 has no oxide film. However, the oxide film may be disposed in the other RF electrode. In this manner, corrosion resistance of the other RF electrode can be improved.

In the above-described exemplary embodiment, a case has been described as an example where retractable mechanism 90 allows first stimulation provider 50 to retract with respect to second stimulation provider 60. However, the retractable mechanism may allow the second stimulation provider to retract with respect to the first stimulation provider. In this case, it is desirable that the first stimulation provider is fixed to the housing. Even in this case, both first stimulation provider and second stimulation provider can be reliably in close contact with skin S.

Alternatively, the present disclosure includes a form obtained by adopting various modifications which are conceivable by those skilled in the related art for each exemplary embodiment, or a form realized by optionally combining the configuration elements and functions in each exemplary embodiment within the scope not departing from the gist of the present disclosure.

In this way, the present disclosure is applicable to the beauty apparatus used for the skin.

## Claims

1. A beauty apparatus (10) comprising:
a first stimulation provider (50) configured to provide a skin with a first stimulation;
a second stimulation provider (60) including a pair of RF electrodes and configured to provide the skin with a second stimulation which is a stimulation induced by a Radio Frequency (RF) and different from the first stimulation; and
a housing (20) that holds the first stimulation provider (50) and the second stimulation provider (60), the second stimulation provider (60) being located in vicinity of the first stimulation provider (50); and
wherein the housing (20) fixes one of the first stimulation provider (50) and the second stimulation provider (60), and
**characterized in that**
the beauty apparatus (10) comprises a retractable mechanism (90) in which the other of the first stimulation provider (50) and the second stimulation provider (60) is retracted from the one of the first stimulation provider (50) and the second stimulation provider (60).

2. The beauty apparatus (10) of claim 1 wherein at least one of the pair of RF electrodes (51, 61) is a metal body whose surface has an oxide film (512), and
wherein the pair of RF electrodes (51, 61) is supplied with a voltage having a frequency bandwidth of 0.3 MHz or higher.

3. The beauty apparatus (10) of claim 1 or 2
wherein the housing (20) includes a first holder (70) and a second holder (80), the first holder (70) holding the first stimulation provider (50), the second holder (80) holding the second stimulation provider (60) and being freely detachable from the first holder (70).

4. The beauty apparatus (10) of claim 3
wherein the second holder (80) has an annular shape and surrounds the first stimulation provider (50), and a portion of an edge portion of the second holder (80) has a flat shape.

5. The beauty apparatus (10) of claim 3 or 4 further comprising:
a terminal (100) for supplying power to the second stimulation provider (60),
wherein the second stimulation provider (60) and the terminal (100) are electrically connected to each other at a plurality of locations.

6. The beauty apparatus (10) of any one of claims 1 to 5, further comprising:
a heater (53) configured to heat the skin from a surface of the skin.

## Patentansprüche

1. Kosmetikgerät (10), umfassend:
einen ersten Stimulator (50), der dazu eingerichtet ist, eine Haut mit einer ersten Stimulation zu versorgen;
einen zweiten Stimulator (60), der ein Paar HF-Elektroden umfasst und dazu eingerichtet ist, die Haut mit einer zweiten Stimulation zu versorgen, die eine durch eine Hochfrequenz (HF) induzierte Stimulation ist und sich von der ersten Stimulation unterscheidet; und
ein Gehäuse (20), das den ersten Stimulator (50) und den zweiten Stimulator (60) hält, wobei sich der zweite Stimulator (60) in der Nähe des ersten Stimulators (50) befindet; und
das Gehäuse (20) den ersten Stimulator (50) oder den zweiten Stimulator (60) fixiert,
und **dadurch gekennzeichnet, dass**
das Kosmetikgerät (10) einen einziehbaren Mechanismus (90) umfasst, bei dem der andere des ersten Stimulators (50) und des zweiten Stimulators (60) von dem einen des ersten Stimulators (50) und des zweiten Stimulators (60) zurückgezogen wird.

2. Kosmetikgerät (10) nach Anspruch 1,
bei dem wenigstens eines der HF-Elektrodenpaare (51, 61) ein Metallkörper ist, dessen Oberfläche einen Oxidfilm (512) aufweist, und
das Paar von HF-Elektroden (51, 61) mit einer Spannung mit einer Frequenzbandbreite von 0,3 MHz oder höher versorgt wird.

3. Kosmetikgerät (10) nach Anspruch 1 oder 2,
bei dem das Gehäuse (20) einen ersten Halter (70) und einen zweiten Halter (80) umfasst, wobei der erste Halter (70) den ersten Stimulator (50) hält und der zweite Halter (80) den zweiten Stimulator (60) hält und frei von dem ersten Halter (70) lösbar ist.

4. Kosmetikgerät (10) nach Anspruch 3,
bei dem der zweite Halter (80) eine ringförmige Form hat und den ersten Stimulator (50) umgibt und ein Abschnitt eines Randabschnitts des zweiten Halters (80) eine flache Form hat.

5. Kosmetikgerät (10) nach Anspruch 3 oder 4, weiterhin umfassend:
einen Anschluss (100) zum Versorgen des zweiten Stimulators (60) mit Strom, wobei der zweite Stimulator (60) und der Anschluss (100) an mehreren Stellen elektrisch miteinander verbunden sind.

6. Kosmetikgerät (10) nach einem der Ansprüche 1 bis 5, weiterhin umfassend:
eine Heizeinrichtung (53), die dazu eingerichtet ist, die Haut von einer Hautoberfläche zu erwärmen.

## Revendications

1. Appareil d'esthétique (10) comprenant :
un premier fournisseur de stimulation (50) configuré pour fournir une première stimulation à une peau ;
un second fournisseur de stimulation (60) comprenant une paire d'électrodes RF et configuré pour fournir une seconde stimulation à la peau qui est une stimulation induite par une radio fréquence (RF) et différente de la première stimulation ; et
un logement (20) qui maintient le premier fournisseur de stimulation (50) et le second fournisseur de stimulation (60), le second fournisseur de stimulation (60) étant localisé à proximité du premier fournisseur de stimulation (50) ; et
le logement (20) fixant l'un du premier fournisseur de stimulation (50) et du second fournisseur de stimulation (60), et
**caractérisé en ce que**
l'appareil d'esthétique (10) comprend un mécanisme rétractable (90) dans lequel l'autre du premier fournisseur de stimulation (50) et du second fournisseur de stimulation (60) est rétracté de l'un du premier fournisseur de stimulation (50) et du second fournisseur de stimulation (60).

2. Appareil d'esthétique (10) selon la revendication 1,
au moins l'une de la paire des électrodes RF (51, 61) est un corps métallique dont la surface présente un film d'oxyde (512), et
la paire des électrodes RF (51, 61) est alimentée d'une tension ayant une largeur de bande de fréquence de 0,3 MHz ou plus.

3. Appareil d'esthétique (10) selon la revendication 1 ou 2,
le logement (20) comprenant un premier dispositif de maintien (70) et un second dispositif de maintien (80), le premier dispositif de maintien (70) maintenant le premier fournisseur de stimulation (50), le second dispositif de maintien (80) maintenant le second fournisseur de stimulation (60) et étant librement détachable du premier dispositif de maintien (70).

4. Appareil d'esthétique (10) selon la revendication 3,
le second dispositif de maintien (80) présentant une forme annulaire et entourant le premier fournisseur de stimulation (50), et une partie d'une partie de bordure du second dispositif de maintien (80) ayant une forme plate.

5. Appareil d'esthétique (10) selon la revendication 3 ou 4, comprenant en outre :
une borne (100) pour alimenter de l'énergie au second fournisseur de stimulation (60),
le second fournisseur de stimulation (60) et la borne (100) étant électriquement raccordés l'un à l'autre au niveau d'une pluralité de localisations.

6. Appareil d'esthétique (10) selon l'une quelconque des revendications 1 à 5, comprenant en outre :
un dispositif de chauffage (53) configuré pour chauffer la peau depuis une surface de la peau.
